# EUROPÄISCHE PATENTANMELDUNG

(11) **EP 0 588 071 A2**
(43) Veröffentlichungstag der Anmeldung: **23.03.1994**
(21) Anmeldenummer: 93113051.2
(22) Anmeldetag: 14.08.1993
(51) Int. Cl.: A61M 1/36, B04B 5/04

(54) **Zentrifuge zum Trennen von Blut in seine Bestandteile**

(30) Priorität: 21.08.1992 DE 4227695
(71) Anmelder: Fresenius AG, D-61350 Bad Homburg (DE)
(72) Erfinder: Pusinelli, Thomas, D-63674 Altenstadt-Waldsiedlung (DE)
(74) Vertreter: Fuchs, Luderschmidt & Partner Patentanwälte

(57) **Zusammenfassung**

Zentrifuge (10) zum Auftrennen von Blut in seine Bestandteile, insbesondere Single-needle-Zentrifuge, bei der in einem Sammelschritt Erythrozyten und Plasma in einem Speichergefäß (54) zwischengespeichert werden und in einem Rezirkulationsschritt die im Speichergefäß (54) zwischengespeicherten Blutbestandteile erneut durch die Separationskammer (14) geführt und anschließend dem Patienten zurückgegeben werden.

## Beschreibung

Die Erfindung betrifft eine Zentrifuge zum Auftrennen von Blut in seine Bestandteile, mit wenigstens einer Entnahmeeinrichtung für Blut, einer von der Entnahmeeinrichtung abgehenden Zuführleitung, die mit der Zentrifuge verbunden ist und in die eine Blutpumpe eingeschaltet ist, einer ersten von der Zentrifuge abgehenden Abzugsleitung, die mit einem ersten Auffangbehälter verbunden ist und in die eine erste Pumpe eingeschaltet ist, und einer Abzugsleitungsanordnung zum Rückführen der nicht abzutrennenden Blutbestandteile, die mit der Entnahmeeinrichtung verbunden ist. Eine solche Zentrifuge ist beispielsweise aus der DE-A-39 31 471 bekannt.

Blutzentrifugen oder Plasmaphoresevorrichtungen sind beispielsweise aus der DE-OS 26 12 988 bekannt, bei der Blut in einem extrakorporalen Kreislauf mittels einer Nadel entnommen, gleitringdichtungsfrei einer Zentrifuge zugeführt, dort in seine Bestandteile aufgetrennt und anschließend über eine weitere Nadel dem Patienten zurückgegeben wird. Diese Separationsbehandlung wird im wesentlichen kontinuierlich durchgeführt, wobei das Blut in der Zentrifuge im wesentlichen in drei Hauptfraktionen aufgetrennt wird, nämlich die Erythrozytenfraktionen, die auf der Erythrozytenfraktion schwimmende buffy coat (Zellgemisch, insbesondere aus Thrombozyten und Leukozyten) und - radial am weitesten innen liegend - die Plasmafraktion. Der Separationsgrad für die Leukozyten- bzw. Thrombozytenfraktion kann bis zu 70 % betragen, d.h. von den der Zentrifuge zugeführten Leukozyten werden mindestens 30 % wieder zum Patienten zurückgeführt.

Diese 2-Nadelmethode ist für den Patienten jedoch relativ unangenehm. Insofern existiert eine Plasmapheresevorrichtung in Form einer Zentrifuge, bei der die Separation mit nur einer Nadel durchgeführt wird. Derartige Verfahren sind beispielsweise in der DE-A-27 45 041, der US-A-45 31 932, DE-A-39 31 471, EP-A-0 450 737 oder EP-A-0 487 096 beschrieben. Das 1-Nadelverfahren muß jedoch intermittierend durchgeführt werden, d.h. dem Patienten muß in einem ersten Zyklusschritt Blut entnommen und der Zentrifuge zugeführt werden und in einem zweiten Zyklusschritt müssen die zurückzuführenden Blutbestandteile wieder zurückgegeben werden. Dieses Verfahren, bei dem das Blut nur mit einer Nadel entnommen und rückgeführt wird, dauert normalerweise länger als eine 2-Nadel-Separation, ist jedoch wegen der geringen Belastung des Patienten wünschenswert oder sogar notwendig bei verknorpelten oder schlecht zu findenden Venen.

Darüber hinaus ist die Thrombozytenfraktion immer noch mit einem bestimmten Anteil von Lymphozyten bzw. die Leukozytenfraktion mit einem Teil an Erythrozyten und Thrombozyten verunreinigt, so daß ein höherer Reinheitsgrad erwünscht ist.

Zur Verbesserung des Abtrenn- und Reinheitsgrades schlägt beispielsweise die DE-A-39 31 471 eine Rezirkulation bestimmter abgetrennter Blutbestandteile mit unbehandeltem Blut vor, was jedoch nur eine gewisse Verbesserung der Trennwirkung erbringt.

Insofern liegt der Erfindung die Aufgabe zugrunde, ein Verfahren oder eine Vorrichtung der eingangs erwähnten Art zur Verfügung zu stellen, mit der die Ausbeute und die Reinheit der abzutrennenden Blutbestandteile, insbesondere in einer Single-Needle-Zentrifuge, bei möglichst kurzen Behandlungszeiten, verbessert werden.

Die Aufgabe wird dadurch gelöst, daß das Ableitungssystem eine Plasmaabzugsleitung und eine Erythrozytenabzugsleitung aufweist und eine in die Plasmaabzugsleitung eingeschaltete Abzugspumpe vorgesehen ist, von der Plasmaabzugsleitung an einem ersten Verzweigungspunkt eine Plasmaabzweigleitung abzweigt, die mit einem Speichergefäß verbunden ist, von der Erythrozytenabzugsleitung an einem zweiten Verzweigungspunkt eine Erythrozytenabzweigleitung abzweigt, die mit dem Speichergefäß verbunden ist, von dem Speichergefäß eine Sammelleitung abgeht, die mit der Zuführungsleitung an einem dritten Verzweigungspunkt stromauf der Blutpumpe verbunden ist, eine Absperreinrichtung vorgesehen ist, die im Kollektionszyklus die Sammelleitung, die Plasmaabzugsleitung stromauf des ersten Verzweigungspunkts und die Erythrozytenabzugsleitung stromab des zweiten Verzweigungspunkts sperrt und die im nachfolgend erläuterten Rezirkulationszyklus aufgezählten Leitungen offen hält und im Rezirkulationszyklus die Zuführungsleitung stromauf des dritten Verzweigungspunkts, die Plasmaabzweigleitung und die Erythrozytenabzweigleitung sperrt und die im Kollektionszyklus genannten Leitungen offenhält.

Mit der erfindungsgemäßen Zentrifuge wird in einem ersten Zyklusschritt eine vorbestimmte Blutmenge in verschiedene Fraktionen separiert, wobei die erste Fraktion in einem ersten Behälter und die restliche Fraktion in einem Zwischenspeicher gespeichert werden, und in einem zweiten Zyklusschritt die restliche Fraktion einer erneuten Separationsbehandlung unterzogen wird, wobei ein weiterer Teil der ersten Fraktion abgetrennt wird und der restliche Blutbestandteil anschließend zurückgegeben wird.

Durch die erfindungsgemäße Separationsbehandlung wird die Reinheit der gewonnenen Blutbestandteile weiter erhöht, wobei die Zeit der Separation trotz der Rezirkulation minimal klein gehalten wird, da während der Rezirkulationsphase zugleich die Reinfusion erfolgt. Hierdurch ist es möglich, auch während der Rückführung Blutkomponenten zu gewinnen. Außerdem läuft die Zentrifuge kontinuierlich, wodurch zum einen die Separationszeit bei höchstmöglicher Effektivität maximal kurz ist, zum anderen die Anlaufzeit, die zur Ausbildung einer deutlichen Trenngranze benötigt wird, entfällt.

Demzufolge liegen die Vorteile der Erfindung in einer effektiven Trennung der Blutbestandteile durch die nochmalige Rezirkulation des gleichen Bluts in möglichst kurzer Zeit.

Während das Abtrennungsverfahren auch mit einer üblichen 2-Nadelanordnung durchgeführt werden kann, ist jedoch die 1-Nadelanordnung bevorzugt. Insofern weist die Entnahmeeinrichtung eine einzige Nadel auf, die an dem Patienten abgewandten Ende entweder Y-förmig verzweigt ist oder aber im Entnahmeschlauch eine Y-förmige Verzweigung aufweist.

Die während der Kollektionsphase abgezogene und prozessierte Blutmenge hängt im wesentlichen vom Fassungsvermögen des Sammelbehälters, der üblicherweise als Beutel ausgebildet ist, und des Speichergefäßes ab, das ebenfalls als Beutel gebildet ist. Insofern ist diese Blutmenge vorbestimmt und wird durch die Blutpumpe über die Zuführungsleitung dem Patienten oder einer anderen Blutquelle (Blutbeutel) entnommen.

Das entnommene Blut, das üblicherweise mit einem Antikoagulierungsmittel versehen ist, durchläuft die Separationsbehandlung in der Zentrifugen-Separationskammer, in der das Blut in seine einzelnen Fraktionen aufgetrennt wird. Diese bestehen üblicherweise aus der am weitesten außen liegenden Erythrozytenfraktion, auf der die buffy-coat schwimmt. Hieran schließt sich nach innen die Plasmafraktion an. Die Einstellung dieser Fraktionsgrenzen sowie die Abtrennung erfolgt nach üblichen Verfahren, wie sie beispielsweise in der DE-OS 33 01 113 beschrieben ist, auf die hiermit Bezug genommen wird.

Diese Fraktionen werden nach einem vorbestimmten Ablauf aus der Separationskammer abgezogen bzw. aus der Separationskammer durch die Wirkung der Blutpumpe hinaus gefördert. Während der Kollektionsphase fördert die WBC- oder PLT-Pumpe die WBC- oder PLT-Fraktion in einen separaten WBC- oder PLT-Beutel, wahrend die Erythrozytenfraktion durch die Wirkung der Blutpumpe direkt in den Speicherbeutel gefördert wird. Die Plasmafraktion wird mit Hilfe der Abzugspumpe, die als Plasmapumpe ausgebildet ist, in das gleiche Speichergefäß gepumpt. WBC bedeutet "white blood cells" (Leukozyten), während PLT "platelets" (Thrombozyten) und RBC "red blood cells" bedeutet.

Gemäß einer bevorzugten Ausführungsform ist das Speichergefäß so ausgebildet, daß der Boden in der Gebrauchslage eine Schräge bildet. Dabei wird die Plasma-Abzweigleitung so dem Speichergefäß zugeführt, daß sie im unteren schrägen Bereich in den Beutel mündet, während die Erythrozyten (RBC)-Abzweigleitung in den oberen Bereich mündet. Aufgrund des größeren spezifischen Gewichts der RBC findet damit eine bessere Durchmischung von Plasma und RBC innerhalb des Speicherbeutels statt.

In der Kollektionsphase wird somit der WBC-Beutel und das Speichergefäß mit einem bestimmten Volumen angefüllt. Ist insbesondere im Speicherbeutel das vorbestimmte Volumen erreicht, so wird mit Hilfe der Absperreinrichtung eine Veränderung des Fluidlaufs innerhalb des extrakorporalen Leitungssystems vorgenommen. Dieses Leitungssystem ist - wie bei sämtlichen extrakorporalen Blutsystemen - in sich geschlossen und somit gegenüber der Umwelt steril und üblicherweise aus einem polymeren elastischen Material (PVC-Schläuchen und PVC-Beuteln) gebildet. Aufgrund der Elastizität dieser Kunststoffschläuche können externe Klemmen als Absperreinrichtung für die jeweiligen Schlauchabschnitte herangezogen werden. Diese Klemmen können jeweils als Einzelklemmen oder als Schlauchklemmanordnung ausgebildet sein, d.h. aus Klemmpaaren bestehen, die jeweils zwischen zwei Zuständen (Kollektionsphase und Rezirkulationsphase) schaltbar sind und durch ein einziges Steuerorgan betätigt werden. Die letztgenannte Anordnung ist aus Kostengründen bevorzugt.

Nach Ablauf der Kollektionsphase wird in die Rezirkulationsphase umgeschaltet. Dabei wird die Blutzuführungsleitung stromauf des dritten Verzweigungspunktes geschlossen und zugleich wird die von dem Speicherbeutel abgehende Sammelleitung geöffnet. Es werden weiterhin die RBC- und Plasmaabzweigleitung geschlossen, so daß keine weiteren Blutbestandteile mehr dem Speicherbeutel zugeführt werden. Andererseits wird die Plasmaabzugsleitung stromab des ersten Verzweigungspunkts und die RBC-Abzugsleitung stromab des zweiten Verzweigungspunkts geöffnet, so daß RBC und Plasma zum Patienten bzw. Spender zurückgeführt werden können. Dies geschieht mit Hilfe der Plasmapumpe bzw. der Blutpumpe. Die beiden Abzugsleitungen münden vorteilhafterweise in ein Mischgefäß, von dem eine Rückführleitung zum Patienten unmittelbar zurückgeht.

Gemäß einer weiteren Ausführungsform ist stromab des ersten Verzweigungspunkts und der Absperrklemme in der Plasmaabzugsleitung ein vierter Abzweig von der Plasmaabzugsleitung vorgesehen, von dem eine Plasmasammelleitung abgeht, die in einen Plasmabeutel mündet. Stromab dieses vierten Abzweigs ist eine zweite Absperreinrichtung vorgesehen, die wechselweise die Plasmasammelleitung und die Plasmaabzugsleitung absperren kann. Diese kann - wie vorstehend erläutert - aus Einzelabsperreinrichtungen (Klemmanordnung) oder einem Klemmenpaar bestehen. Dabei wird die zweite Absperreinrichtung unabhängig von der ersten Absperreinrichtung betrieben.

Beim zweiten Durchlauf des Bluts wird also erneut eine WBC- oder PLT-Komponente gewonnen. Zusätzlich wird das zum zweiten Mal dem Separationsschritt unterworfene Blut mit der Flußgeschwindigkeit, die durch die Vollblutpumpe vorgegeben ist, vollständig reinfundiert. Dabei wird, um eine unnötige Verdünnung des zu separierenden Bluts und eine übermäßige Reinfusion von Antikoagulanzlösungen zu vermeiden, die Antikoagulanzzufuhr während der Rezirkulationsphase unterbunden.

Ein weiterer Aspekt der Erfindung ist das Einmalschlauchsystem einschließlich der Separationskammer und sämtlicher Speicherbeutel und der nadelförmigen Entnahmeeinrichtung. Ein solches Einmalsystem, das - wie vorstehend erläutert ist - in sich geschlossen gegenüber der Umwelt steril ist, wird als vorgefertigtes Disposable firmenseitig geliefert und kann unmittelbar nach dem Einsetzen in die Zentrifugenantriebsvorrichtung, die nicht Gegenstand der Erfindung ist, zur Anwendung gebracht werden.

Ein Ausführungsbeispiel ist nachstehend anhand der Zeichnung erläutert.

Es zeigen:
- Figur 1: eine allgemeine schematische Skizze der erfindungsgemäßen Vorrichtung,
- Figur 2: die Vorrichtung gemäß Figur 1 in der Kollektionsphase und
- Figur 3: die Vorrichtung gemäß Figur 1 in der Rezirkulationsphase.

Mit 10 ist eine Zentrifugenanordnung gezeigt, die aus einer gestrichelt gezeichneten Zentrifugenantriebsanordnung 12, einer Separationskammer 14, die in der Zentrifugenantriebsanordnung 12 antreibbar ist, und einem Schlauchsystem 16, das mit der Separationskammer 14 verbunden ist, besteht.

Das Schlauchsystem 16 weist eine in einen Patienten oder Blutbeutel einstechbare Nadel 18 auf, von der eine Zuführungsleitung 20 abgeht, die mit der Separationskammer 14 verbunden ist. In diese Zuführungsleitung ist eine Blutpumpe 22 in Form einer peristaltischen Pumpe eingeschaltet.

Von der Separationskammer 14 geht eine WBC-PLT-Leitung 24 ab, deren Ende in einen WBC-PLT-Beutel 26 als ersten Auffangbehälter mündet. In diese WBC-PLT-Leitung 24 ist eine WBC-PLT-Pumpe 28 eingeschaltet.

Desweiteren geht von der Separationskammer 14 eine Plasmaabzugsleitung 30 ab, in die eine Plasmapumpe 32 eingeschaltet ist. Die Plasmaabzugsleitung 30 mündet an ihrem anderen Ende in eine als Sammelkammer ausgebildete Tropfkammer 34.

Schließlich geht von der Separationskammer 14 eine RBC-Abzugsleitung 36 ab, deren Ende ebenfalls in die Tropfkammer 34 mündet.

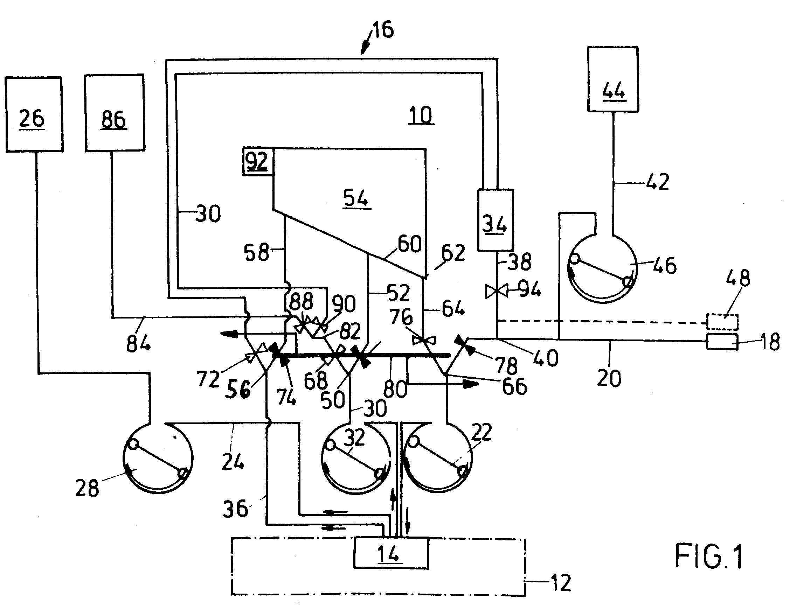

Von der Tropfkammer 34 geht schließlich eine Rückführleitung 38 ab, die entweder unmittelbar mit der dann Y-förmig ausgebildeten Nadel 18 verbunden ist oder - wie in Figur 1 gezeigt ist - benachbart zur Nadel 18 in die Zuführungsleitung 20 am Punkt 40 mündet.

Mit der Zuführungsleitung 20 ist weiterhin eine Antikoagulanzleitung 42 verbunden, die von einem Antikoagulanz enthaltenden Behälter 44 abgeht und in die eine Antikoagulanzpumpe 46 eingeschaltet ist.

Gemäß einer zweiten Ausführungsform ist - wie in Figur 1 gestrichelt dargestellt ist - die Rückführleitung 38 nicht mit der Zuführungsleitung 20, sondern vielmehr mit einer zweiten Nadel 48 verbunden.

Von der Plasmaabzugsleitung 30 geht stromab der Plasmapumpe 32 am ersten Verzeigungspunkt 50 eine Plasmaabzweigleitung 52 ab, die in einem Speicherbeutel 54 mündet.

Von der RBC-Abzugsleitung 36 geht von einem zweiten Verzweigungspunkt 56 eine RBC-Abzweigleitung 58 ab, die ebenfalls in den Speicherbeutel 54 mündet.

Dieser Speicherbeutel 54 weist - wie in der Figur 1 gezeigt - vorzugsweise eine schräg verlaufende untere Kante 60 auf. Dabei mündet die RBC-Abzweigleitung 58 oberhalb der Plasmaabzweigleitung 52 in den Speicherbeutel 54. Dies hat den Vorteil, daß die spezifisch schwereren RBC sich leichter mit dem spezifisch leichteren Plasma vermischen lassen, so daß keine Separierung des Bluts im Speicherbeutel 54 in seine Bestandteile stattfinden kann.

Schließlich geht von Speicherbeutel 54, und zwar vom untersten Ende 62, eine Sammelleitung 64 ab, die an einem dritten Abzweigpunkt 66 mit der Zuführungsleitung 20 verbunden ist. Dieser dritte Abzweigpunkt 66 liegt stromauf der Blutpumpe 22 bzw. zwischen der Blutpumpe 22 und dem Einmündungspunkt 40.

Stromab des ersten Verzweigungspunkts 50 ist in die Plasmaabzugsleitung 30 eine erste Klemme 68 und in die Plasmaverzweigungsleitung 52 eine zweite Klemme 70 eingeschaltet. Die beiden Klemmen 68 und 70 bilden ein erstes Klemmenpaar.

Weiterhin ist stromab des zweiten Verzweigungspunkts 56 in die RBC-Abzugsleitung 36 eine dritte Klemme 72 und in die RBC-Verzweigungsleitung 58 eine vierte Klemme 74 eingeschaltet. Diese beiden Klemmen 72 und 74 bilden das zweite Klemmenpaar.

Schließlich ist stromauf des dritten Verzweigungspunkts 66 in die Sammelleitung 64 eine fünfte Klemme 76 und die Zuführungsleitung 20 zwischen Blutpumpe 22 und Mündungspunkt 40 eine sechste Klemme 78 eingeschaltet. Diese Klemmen 76 und 78 bilden ein drittes Klemmenpaar.

Das erste, zweite und dritte Klemmenpaar sind über eine Klemmensteuerungsvorrichtung 80 miteinander derart verbunden, daß jeweils die erste, dritte und fünfte Klemme geschlossen sind, wenn die zweite, vierte und sechste Klemme geöffnet sind bzw. nach Umschaltung der Klemmsteuerungsvorrichtung 80 geöffnet wird, so daß die zweiten, vierten und sechsten Klemmen geschlossen sind.

Die Klemmen 68-78 können entweder starr über eine Klemmsteuerungsvorrichtung 80 wechselweise geschlossen bzw. geöffnet werden. Sie können jedoch aber auch ohne eine solche mechanische Klemmsteuerung vorliegen, so daß sie unmittelbar separat über eine nicht gezeigte elektrische Steuerungsvorrichtung betätigbar sind.

Stromab der ersten Klemme 68 geht gemäß einer bevorzugten Ausführungsform von der Plasmarückführleitung 38 an einem vierten Verzweigungspunkt 82 eine Plasmasammelleitung 84 ab, die in einen Plasmasammelbeutel 86 mündet. Stromab des Verzweigungspunkts 82 ist eine siebte Klemme 88 in die Plasmasammelleitung 84 eingeschaltet, während ebenfalls stromab des vierten Verzweigungspunkts 82 in die Plasmarückführleitung 30 eine achte Klemme 90 eingeschaltet ist, wobei die siebte und achte Klemme 88, 90 ein viertes Klemmenpaar bilden. Dieses vierte Klemmenpaar ist unabhängig von den Klemmen 68-78, schaltet jedoch wechselweise eine der beiden Leitungen in den geöffneten Zustand.

Der Füllzustand des Speicherbeutels 54 wird vorteilhafterweise mit einem Füllstandsdetektor 92 bestimmt, der als Gewichts- oder Füllstandssensor ausgeführt sein kann und die in den Speicherbeutel 54 eingeführte Blutmenge gewichtsmäßig oder volumenmäßig bestimmt und nach Erreichen eines vorbestimmten Werts einen Schaltvorgang auslöst.

Im übrigen ist an der Rückführleitung 38 eine Absperranordnung 94 vorgesehen, um die Anordnung in den sicheren Zustand überführen zu können.

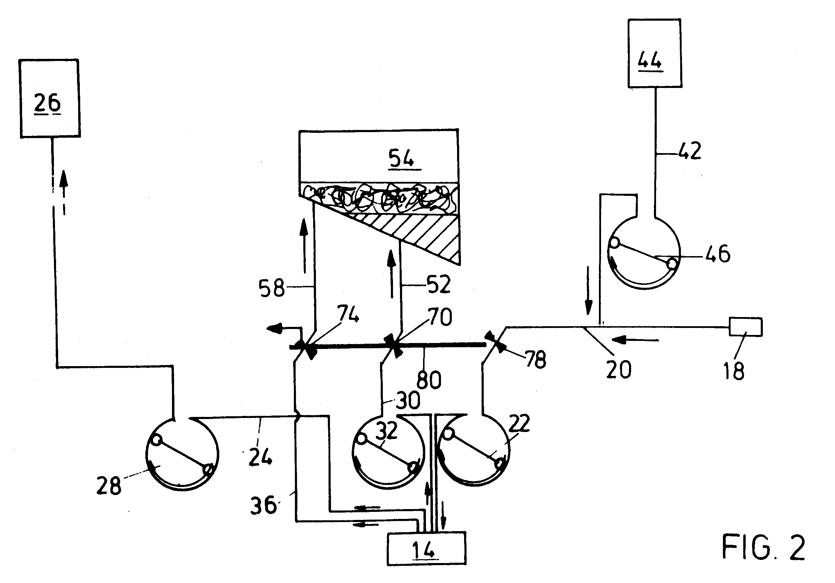

In Figur 2 ist die Zentrifugenvorrichtung 10 gemäß Figur 1 in der Kollektionsphase dargestellt, wobei sämtliche nicht aktivierten bzw. nicht in der Kollektionsphase eingesetzten Vorrichtungsteile aus Vereinfachungsgründen nicht gezeigt sind. Dabei sind die geöffneten Klemmen 70, 74 und 78 voll geschwärzt dargestellt.

In der Kollektionsphase wird Blut über die Nadel 18 und die Zuführungsleitung 20 mit Hilfe der Blutpumpe 22 der Separationskammer 14 zugeführt. Dabei wird Antikoagulationslösung aus dem Beutel 44 und die Leitung 42 mit Hilfe der Antikoalanzpumpe 46 in die Zuführungsleitung 20 eingeführt, wo eine Vermischung mit Blut stattfindet.

In der Separationskammer 14 erfolgt nach einem vorher bestimmten Trennverfahren, wie es beispielsweise in der DE-OS 33 01 113 beschrieben ist, die Aufteilung des Bluts in seine Bestandteile, wobei mit Hilfe der Plasmapumpe 32 Plasma über die Plasmaabzugsleitung 30 in den Speicherbeutel 54 gepumpt wird. WBC und PLT werden über die WBC-PLT-Abzugsleitung 24 mit Hilfe der WBC-PLT-Pumpe 28 in den WBC-PLT-Beutel 26 überführt. Die in der Separationskammer 14 weiterhin vorliegende RBC-Schicht wird durch das zugeführte Blut über die RBC-Abzugsleitung 36 in den Speicherbeutel 54 verdrängt und durchmischt sich dort mit dem Plasma. Sobald eine vorbestimmte Menge Blut im Speicherbeutel 54 vorliegt, wird vom Detektor 92 ein Schaltvorgang ausgelöst. Andererseits kann aber auch die Blutpumpe 22 nach Erreichen einer bestimmten Fördermenge einen solchen Schaltvorgang auslösen.

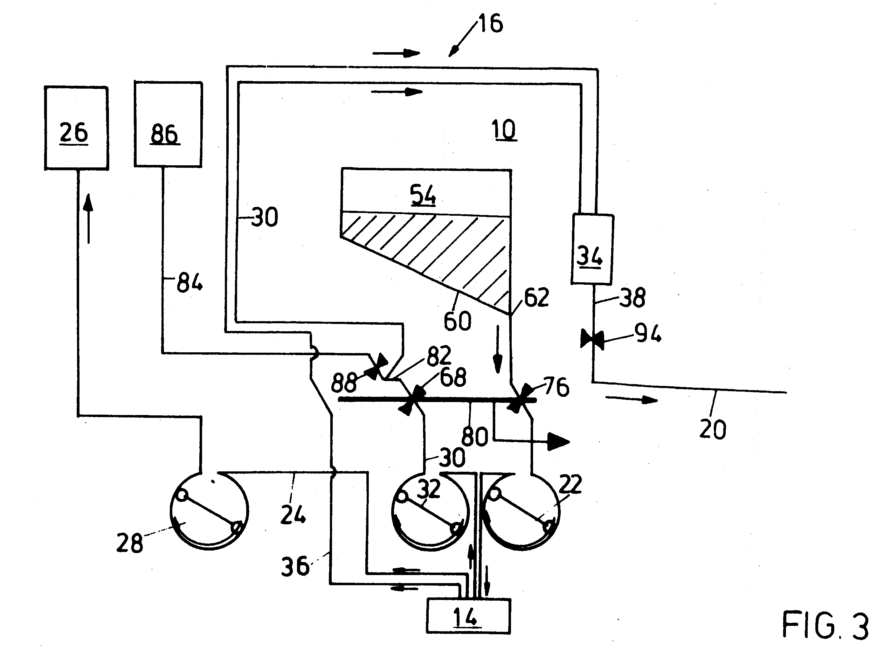

Dieser Schaltvorgang schaltet die Klemmsteuerung 80 von der Kollektionsphase, wie sie in Figur 2 dargestellt ist, in die Rezirkulationsphase, wie sie in Figur 3 dargestellt ist. In dieser Figur sind ebenfalls aus Übersichtlichkeitsgründen diejenigen Vorrichtungsteile weggelassen worden, die in dieser Phase nicht benötigt werden bzw. desaktiviert sind.

In der Rezirkulationsphase sind die erste, dritte und fünfte Klemme 68, 72 und 76 geöffnet, d.h. die Blutpumpe 22 pumpt nunmehr nicht mehr unmittelbar Blut von der Blutquelle ab, sondern pumpt Blut aus dem Speicherbeutel 54 erneut in die Separationskammer 14, um einen zweiten Separationsvorgang an dem nahezu gleichen Blutvolumen auszulösen.

Nach der erneuten Auftrennung des Bluts, was zu einer Verbesserung des WBC-Abtrenngrads bis zu 90 % führen kann, werden RBC über die RBC-Abzugsleitung 36 und Plasma über die Plasmaabzugsleitung 30 bis zur Tropfkammer 34 überführt, in der eine intensive Durchmischung dieser beiden Bestandteile stattfindet. Von dort gelangt das Blut über die Rückführleitung 38 in die nunmehr als Rückführleitung dienende Zuführungsleitung 20 zur Nadel 18 und von dort zum Patienten oder zum Blutbeutel zurück.

Während dieser Reinfusionsphase werden - wie vorstehend erläutert - WBC und PLT über die WBC-PLT-Leitung 24 in den WBC-PLT-Beutel 26 gepumpt.

In einer bevorzugten Ausführungsform kann während dieser zweiten Phase auch Plasma gesammelt werden, das in dem Plasmasammelbeutel 86 gespeichert wird. Hierzu wird das vierte Klemmenpaar 80, 90 wechselweise betätigt, d.h. es wird eine der Klemmen 88 bzw. 90 geöffnet bzw. geschlossen. Dabei wird nur soviel Plasma entnommen, daß die Rückführung der ansonsten zu viskosen RBC nicht gestört wird.

Die Klemme 94 wird üblicherweise während der Kollektionsphase geschlossen und während der Reinfusionsphase geöffnet. Sie muß jedoch aber auch jederzeit geschlossen werden können (beispielsweise in Alarmfällen), so daß diese Klemme 94 nicht mit einem starren Ventiltrieb, wie er in der Ventilsteuerungsvorrichtung 80 vorgegeben ist, verbunden ist.

Nach Entleerung des Speicherbeutels 54, was wiederum durch den Detektor 92 oder aber die Steuerung der Separationskammer 14, wie in der vorstehenden DE-OS 33 01 113 dargestellt, festgestellt werden kann, wird erneut ein Schaltvorgang ausgelöst, so daß wiederum die Kollektionsphase gestartet wird.

## Patentansprüche

1. Zentrifuge (10) zum Auftrennen von Blut in seine Bestandteile, mit wenigstens einer Entnahmeeinrichtung (18) für Blut, einer von der Entnahmeeinrichtung abgehenden Zuführleitung (20), die mit der Zentrifuge (10) verbunden ist und in die eine Blutpumpe (20) eingeschaltet ist, einer ersten von der Zentrifuge (10) abgehenden Abzugsleitung (24), die mit einem ersten Auffangbehälter (26) verbunden ist und in die eine erste Pumpe (28) eingeschaltet ist, und einer Abzugsleitungsanordnung (16, 30, 38) zum Rückführen der nicht abzutrennenden Blutbestandteile, die mit der Entnahmeeinrichtung (18) verbunden ist, dadurch gekennzeichnet, daß die Abzugsleitungsanordnung eine Plasmaabzugsleitung (30) und eine Erythrozytenabzugsleitung (36) aufweist, eine in die Plasmaabzugsleitung (30) eingeschaltete Abzugspumpe (32) vorgesehen ist, von der Plasmaabzugsleitung (30) an einem ersten Verzweigungspunkt (50) eine Plasmaabzweigleitung (52) abzweigt, die mit einem Speichergefäß (54) verbunden ist, von der Erythrozytenabzugsleitung (36) an einem zweiten Verzweigungspunkt (56) eine Erythrozytenabzweigleitung (58) abzweigt, die mit dem Speichergefäß (54) verbunden ist, von dem Speichergefäß (54) eine Sammelleitung (64) abgeht, die mit der Zuführungsleitung (20) an einem dritten Verzweigungspunkt (66) stromauf der Blutpumpe (22) verbunden ist, und eine Absperreinrichtung vorgesehen ist, die im Kollektionszyklus die Sammelleitung (64), die Plasmaabzugsleitung (30) stromab des ersten Verzweigungspunkts (50) und die Erythrozytenabzugsleitung stromab des zweiten Verzweigungspunkts (56) sperrt und die im nachfolgend genannten Rezirkulationszyklus aufgezählten Leitungen offen hält und im Rezirkulationszyklus die Zuführungsleitung (20) stromauf des dritten Verzweigungspunkts (66), die Plasmaabzweigleitung (52) und die Erythrozytenabzweigleitung (58) sperrt und die im Kollektionszyklus genannten Leitung offenhält.

2. Zentrifuge nach Anspruch 1, dadurch gekennzeichnet, daß zu der Absperreinrichtung eine erste bzw. zweite Klemme (68, 70), die stromab des ersten Verzweigungspunkts (50) in der Plasmaleitung (30) bzw. der Plasmaverzweigungsleitung (52) angeordnet sind, als erstes Klemmenpaar, eine dritte bzw. vierte Klemme (72, 74), die stromab des zweiten Verzweigungspunkts (56) in der Erythrozytenabzugsleitung (36) bzw. der Erythrozytenverzweigungsleitung (58) angeordnet sind, als zweites Klemmenpaar, und eine fünfte bzw. sechste Klemme (76, 78), die stromauf des dritten Verzweigungspunkts (66) in der Sammelleitung (64) bzw. der Zuführungsleitung (20) angeordnet sind, als drittes Klemmenpaar gehören, wobei die erste, dritte und fünfte Klemme (68, 72, 76) gegensinnig zu der zweiten, vierten und sechsten Klemme (70, 74, 78) angesteuert werden.

3. Zentrifuge nach Anspruch 2, dadurch gekennzeichnet, daß die Klemmenpaare (68, 79; 72, 74; 76, 78) durch eine starre Klemmensteuervorrichtung (80) betätigbar sind.

4. Zentrifuge nach einem der Ansprüche 1 bis 3, dadurch gekennzeichnet, daß das Speichergefäß (54) einen schräg verlaufenden Boden (60) aufweist und die Erythrozytenabzweigleitung (58) oberhalb der Plasmaabzweigleitung (52) in das Speichergefäß (54) mündet und die Sammelleitung (64) vom tiefsten Punkt (62) des Speichergefäßes (54) abgeht.

5. Zentrifuge nach einem der Ansprüche 1 bis 4, dadurch gekennzeichnet, daß stromab der ersten Klemme (68) von der Plasmarückführleitung (38) an einem vierten Verzweigungspunkt (82) eine Plasmasammelleitung (84) abgeht, die in einen Plasmasammelbeutel (86) mündet, und daß stromab des vierten Verzweigungspunkts (82) eine vierte Klemmenanordnung (88, 90) vorgesehen ist, die wechselweise die Plasmarückführleitung (30) und die Plasmasammelleitung (84) in vorbestimmter Taktfrequenz öffnet bzw. schließt.

6. Zentrifuge nach Anspruch 4, dadurch gekennzeichnet, daß der Füllzustand des Speichergefäßes (54) mit einem Füllstandsensor (92) feststellbar ist, der nach Erreichen eines vorbestimmten Werts die Absperreinrichtung umschaltet.

7. Zentrifuge nach einem der Ansprüche 1 bis 6, dadurch gekennzeichnet, daß die Erythrozytenabzugsleitung (36) und die Plasmaabzugsleitung (30) in eine Sammelkammer (34) münden, von der die Rückführleitung (38, 20) abgeht.
